# FASCICULE DE BREVET EUROPEEN

(11) **EP 2 061 746 B1**
(45) Date de publication et mention de la délivrance du brevet: **21.08.2013**
(21) Numéro de dépôt: 07803845.2
(22) Date de dépôt: 05.07.2007
(51) Int. Cl.: C07C 67/20, C07C 67/22, C07C 69/34, C07C 69/44

(54) **PROCEDE DE FABRICATION DE DIESTERS**
VERFAHREN ZUR HERSTELLUNG VON DIESTERN
PROCESS FOR THE PREPARATION OF DIESTERS

(30) Priorité: 18.07.2006 FR 0606510
(43) Date de publication de la demande: 27.05.2009
(73) Titulaire: Rhodia Opérations, 93306 Aubervilliers (FR)
(72) Inventeur: LECONTE, Philippe, 68150 Ribeauville (FR); MARION, Philippe, F-VERNAISON 69390 (FR); JACQUOT, Roland, F-69340 Francheville (FR)
(74) Mandataire: Jacobson, Claude
(86) Numéro de dépôt international: PCT/FR2007/001140
(87) Numéro de publication internationale: WO 2008/009792

(56) Documents cités:
- WO-A1-2007/101929
- DE-C- 730 518
- US-A- 2 548 025
- US-A- 3 567 749
- US-A- 4 408 067
- US-A1- 2002 173 433
- XUE, C.; LUO, F.-T.: "Transformation of amides into esters by the use of chlorotrimethylsilane" JOURNAL OF THE CHINESE CHEMICAL SOCIETY, vol. 51, no. 2, 2004, pages 359-362, XP008077858 TAIPEI, TAIWAN
- LAECKMANN D ET AL: "Synthesis and biological evaluation of aroylguanidines related to amiloride as inhibitors of the human platelet Na<+>/H<+> exchanger" BIOORGANIC AND MEDICINAL CHEMISTRY 2002 UNITED KINGDOM, vol. 10, no. 6, 2002, pages 1793-1804, XP002429666 ISSN: 0968-0896

## Description

La présente invention concerne un procédé de fabrication de diesters à partir de composés imides ou dinitriles.

Elle se rapporte plus particulièrement à un procédé de fabrication de composés diesters à partir de composés dinitriles mettant en oeuvre une hydrolyse en phase vapeur de composés dinitriles.

Elle concerne encore plus particulièrement un procédé de fabrication de diesters à partir de composés dinitriles ramifiés tels que le méthylglutaronitrile ou les composés dinitriles ramifiés obtenus comme sous produits dans le procédé de fabrication de l'adiponitrile par hydrocyanation du butadiène.

Les solvants oxygénés à base de diesters sont de plus en plus utilisés en remplacement d'autres solvants hydrocarbonés, chlorés ou oxygénés plus agressifs pour l'environnement.

En effet, les solvants diesters tels que ceux commercialisés sous le nom connu de Rhodia Solv RDPE obtenus à partir d'un mélange d'acide adipique, acide glutarique et acide succinique présentent l'avantage d'avoir un profil toxicologique très favorable et sont biodégradables et facilement recyclables. Il a également été proposé dans la demande de brevet français non publiée n° 0602011 des composés diesters obtenus à partir de composés ramifiés et plus particulièrement d'un mélange de méthyl-glutaronitrile, éthyl-succinonitrile et adiponitrile.

Dans ce brevet un procédé de fabrication a été décrit, consistant à faire réagir les composés dinitriles avec un alcool en présence d'un acide minéral suivi par une hydrolyse. Ce procédé est connu sous le nom de réaction de PINNER.

Toutefois, un sel d'ammonium est obtenu comme sous-produit dans ce procédé.

US2002/0173433 décrit la préparation de diesters à partir de 2-méthylglutaronitrile en présence d'un catalyseur acide, sans la formation d'imides comme composés intermédiaires. Le procédé décrit dans ce document ne comprend pas l'utilisation d'un catalyseur solide.

Un des buts de la présente invention est de proposer un procédé de fabrication de diesters à partir de composés dinitriles ne présentant pas les inconvénients des procédés de l'art antérieur et notamment ne générant pas d'effluents ou sous-produits importants et éventuellement nocifs pour l'environnement.

A cet effet, l'invention a pour objet un procédé de fabrication en deux étapes de composés diesters par réaction entre un composé imide de formule générale (I) suivante : 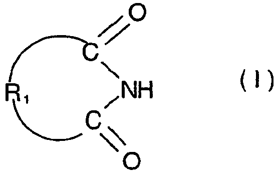 Dans laquelle : R₁ est un radical divalent hydrocarboné comprenant de 2 à 12 atomes de carbones, linéaires ou ramifiés avec un alcool de formule générale II suivante :

R₂ - OH (II)

Dans laquelle R₂ est un radical hydrocarboné pouvant comprendre des hétéroatomes, linéaires ou ramifiés, aliphatique, cycloaliphatique, aromatique ou arylalkyl comprenant de 1 à 20 atomes de carbone.

Le composé imide de formule (I) est obtenu par hydrolyse cyclisante de composés dinitriles de formule générale III suivante :

NC-R₁-CN (III)

dans laquelle R1 a la signification indiquée ci-dessus.

Cette réaction d'hydrolyse cyclisante est réalisée en phase vapeur en présence d'un catalyseur solide

Le composé imide obtenu par hydrolyse du composé dinitrile peut être avantageusement séparé du milieu réactionnel et purifié par les techniques usuelles. Toutefois, il est également possible d'utiliser directement comme réactif dans l'étape de réaction avec un alcool, le milieu réactionnel obtenu après l'étape d'hydrolyse, sans séparation ni purification.

Le catalyseur solide utilisé par la réaction d'hydrolyse cyclisante est choisi dans le groupe comprenant les oxydes métalliques tels que l'alumine, l'oxyde de titane, les hétéropolyacides, les zéolithes de type pentasil et faujasite, les argiles, les phosphates métalliques, les mélanges silice/alumine et analogues

Ainsi, les argiles convenables pour l'invention sont notamment des phyllosilicates qui sont classés par groupes selon leur nature et leurs propriétés physico-chimiques, groupes parmi lesquels on peut citer les kaolins, les serpentines, les smectites ou montmorillonites, les illites ou micas, les glauconites, les chlorites ou vermiculites, les attapulgites ou sépiolites, les argiles à couches mixtes, les allophanes ou imogolites et les argiles à haute teneur en alumine.

Certaines argiles possèdent une structure lamellaire à réseau expansible. Elles présentent la particularité d'adsorber divers solvants, notamment l'eau, entre les feuillets qui les composent, ce qui provoque un gonflement du solide par suite de l'affaiblissement des liaisons électrostatiques entre les feuillets. Ces argiles appartiennent essentiellement au groupe des smectites (ou encore groupe de la montmorillonite) et pour certaines d'entre elles au groupe des vermiculites.

Leur structure est composée de feuillets « élémentaires » à trois couches : deux couches simples de tétraèdres SiO₄ dans lesquelles une partie du silicium peut être remplacée par d'autres cations en position tétraédrique tels que Al³⁺ ou éventuellement Fe³⁺, et entre ces deux couches de tétraèdres, une couche d'octaèdres d'oxygènes au centre desquels se situent des cations métalliques tels que Al³⁺, Fe³⁺, Mg²⁺. Cette couche octaédrique est constituée d'un empilement compact d'oxygènes provenant soit des sommets des tétraèdres précédents soit de groupes hydroxyles OH. Le réseau hexagonal compact de ces oxygènes contient 6 cavités octaédriques.

Lorsque les cations métalliques occupent 4 de ces cavités (2 cavités sur 3 comme dans le cas de l'aluminium par exemple), la couche est dite dioctaédrique; lorsqu'ils occupent toutes les cavités (3 cavités sur 3 comme dans le cas du magnésium par exemple), la couche est dite trioctaédrique.

Les feuillets élémentaires de ces argiles sont porteurs de charges négatives qui sont compensées par la présence de cations échangeables, alcalins tels que Li⁺, Na⁺, K⁺, alcalino-terreux tels que Mg²⁺, Ca²⁺, et éventuellement l'ion hydronium H₃O⁺. Les smectites ont des densités de charge sur les feuillets inférieures à celles des argiles du type vermiculites : environ 0,66 charges par maille élémentaire contre 1 à 1,4 charges par maille élémentaire pour les vermiculites.

Les cations de compensation sont essentiellement le sodium et le calcium dans les smectites, le magnésium et le calcium dans les vermiculites. Du point de vue des densités de charges, smectites et vermiculites sont intermédiaires entre le talc et la pyrophyllite d'une part, dont les feuillets sont neutres et les micas d'autre part, caractérisés par une densité de charges importante sur les feuillets (environ 2 par maille élémentaire) compensée généralement par des ions K⁺.

Les cations interfoliaires des smectites et des vermiculites peuvent être assez facilement remplacés par échange ionique par d'autres cations tels, par exemple, des ions ammonium ou des ions de métaux alcalino-terreux ou de métaux de terres rares.

Les propriétés de gonflement des argiles dépendent de divers facteurs dont la densité de charge et la nature du cation de compensation.

Ainsi les smectites dont la densité de charge est plus faible que celle des vermiculites présentent des propriétés gonflantes nettement supérieures à celles de ces dernières, et constituent donc une classe très intéressante de solides. La distance répétitive ou espacement basal représente la distance la plus courte séparant deux motifs cristallographiquement identiques situés dans deux feuillets adjacents. L'espacement basal des smectites peut ainsi atteindre par gonflement des valeurs allant de 1 nm environ à plus de 2 nm.

Parmi les silicates phylliteux « gonflants » du type smectites, on peut citer les principaux solides suivants de formule générale :

(M₁n+)x/n (M₂)₂VI (M₃)₄IV O₁₀ (OH)₂

où M₁ est le cation interfoliaire
M₂ est le métal en position octaédrique
M₃ est le métal en position tétraédrique
x est le nombre de charges apportées par le cation M₁
Les smectites dioctaédriques
montmorillonite (H, Na, Ca_{1/2})ₓ (MgₓAl₂₋ₓ)VI Si₄IV O₁₀ (OH)₂
beidellite (H, Na , Ca_{1/2})ₓ Al₂VI (AlₓSi₄₋ₓ)IV O₁₀ (OH)₂
nontrolite (H, Na , Ca_{1/2}....)ₓ (Fe, Al)₂VI (AlₓSi₄₋ₓ)IV O₁₀ (OH)₂
Les smectites trioctaédriques
hectorite Naₓ (LiₓMg₃₋ₓ)VI· Si₄IV O₁₀ (OH)₂
saponite Naₓ Mg₃VI (AlₓSi₄₋ₓ)IV O₁₀ (OH)₂
stevensite Na₂ₓ Mg₃₋ₓVI Si₄IV O₁₀ (OH)₂

Après adsorption à saturation d'eau ou d'un solvant polaire organique dans une smectite, l'espacement interfoliaire (entre deux feuillets) est maximal. Il peut atteindre une valeur voisine de 1 nm.

Ces solides sont donc potentiellement intéressants en catalyse car leur surface spécifique et leur acidité potentielles sont élevées.

Selon un mode préférentiel de l'invention, l'argile qui constitue le catalyseur de cyclisation des esters ou amides d'acide 6-aminocaproïque en lactame est une smectite. Plus préférentiellement l'argile est la montmorillonite.

Certaines argiles ont malheureusement l'inconvénient de perdre leur caractère expansé par chauffage à 100°C et de ce fait de ne pas conserver l'augmentation de surface spécifique résultant de leur expansion. C'est le cas notamment des smectites.

Différentes méthodes ont été décrites dans l'art antérieur pour introduire entre les feuillets des smectites des piliers ou ponts pour obtenir des smectites pontées qui conservent un espacement interfoliaire élevé après avoir été soumises à un traitement thermique.

Une méthode consistant à introduire des ponts constitués par des oligomères d'un hydroxyde d'un métal, notamment d'hydroxyde d'aluminium, a été décrite par LAHAV, SHAMI et SHABTAI dans Clays and Clays Mineral, vol.26 (n°2), p. 107-115 (1978) et dans le brevet français 2.394.324. La formation de ponts constitués d'oligomères d'hydroxydes mixtes de silicium et de bore, est décrite dans le brevet US 4.248.739. Une technique de pontage des smectites, par dialyse, à l'aide d'hydroxydes d'aluminium, de chrome, de zirconium et titane etc...est revendiquée dans le brevet EP 0.073.718.

Ces méthodes consistent dans leur principe à mettre l'argile au contact d'une solution contenant des espèces ioniques plus ou moins oligomérisées du type hydroxy-aluminique (dans le cas de l'aluminium). Cette opération est réalisée généralement en solution peu concentrée, à température inférieure à 80°C et si possible en l'absence de trouble constitué par un début de précipitation de l'hydroxyde métallique. Les concentrations de l'ion métallique et de l'argile doivent être optimisées pour qu'il y ait formation de suffisamment de piliers solides et que la porosité de l'argile ne soit pas fortement diminuée par l'insertion d'une trop grande quantité d'oxyde métallique.

Lorsque les ions interfoliaires alcalins ou alcalino-terreux sont remplacés par des protons soit directement à l'aide d'une solution très diluée, soit de préférence par échange avec un sel d'ammonium suivi d'une calcination entre 300 et 700°C, les smectites pontées acquièrent une acidité forte quoique inférieure globalement à celles des zéolithes classiques de type Y ou mordenite par exemple.

Selon une variante particulière de l'invention, le catalyseur peut comporter, outre une argile, un ou plusieurs autres composés métalliques, souvent appelés dopants, tels que par exemple des composés de chrome, de titane, de molybdène, de tungstène, de fer, de zinc. Parmi ces dopants les composés de chrome et/ou de fer et/ou de titane sont considérés comme les plus avantageux.

Ces dopants représentent habituellement, en poids par poids d'argile, de 0 % à 10 % et de préférence de 0 % à 5 %.

Par composé métallique on entend aussi bien l'élément métal que l'ion métallique ou toute combinaison comprenant l'élément métal.

Une autre classe de catalyseur préféré de l'invention consiste dans un catalyseur particulaire obtenu par mise en forme d'au moins un oxyde minéral simple ou mixte d'au moins un élément choisi dans le groupe consistant en le silicium, l'aluminium, le titane, le zirconium, le vanadium, le niobium, le tantale, le tungstène, le molybdène, le fer, les terres rares. Ces oxydes peuvent se trouver sous une forme amorphe ou cristalline.

Selon l'invention, le catalyseur particulaire comprend au moins une macroporosité caractérisé par un volume poreux correspondant aux pores de diamètre supérieur à 500 Å, supérieur ou égal à 5ml/100g.

Cette macroporosité est formée avantageusement pendant le procédé de mise en forme des particules par des techniques décrites ci-dessous ou comme, par exemple, l'addition de porogène. Le catalyseur peut être mis en oeuvre sous diverses formes telles que billes, concassés, extrudés en forme de granulés cylindrique creux ou pleins, de nid d'abeille, pastilles, la mise en forme pouvant éventuellement être réalisée à l'aide d'un liant.

Il peut s'agir tout d'abord de billes d'oxydes minéraux issues d'une mise en forme par oil-drop (ou coagulation en gouttes). Ce type de billes peut par exemple être préparé par un procédé similaire à celui décrit pour la formation de billes d'alumine dans les brevets EP-A-0 015 801 ou EP-A-0 097 539. Le contrôle de la porosité peut être réalisé en particulier, selon le procédé décrit dans le brevet EP-A-0 097 539, par coagulation en gouttes d'une suspension, d'une dispersion aqueuse d'oxyde minéral.

Les billes peuvent être également obtenues par le procédé d'agglomération dans un drageoir ou tambour tournant. Il peut aussi s'agir d'extrudés d'oxydes minéraux. Ceux-ci peuvent être obtenus par malaxage, puis extrusion d'une matière à base de l'oxyde minéral. Le contrôle de la porosité de ces extrudés peut être réalisé par le choix de l'oxyde mis en oeuvre et par les conditions de préparation de cet oxyde ou par les conditions de malaxage de cet oxyde avant extrusion. L'oxyde minéral peut ainsi être mélangé lors du malaxage à des porogènes. A titre d'exemple, les extrudés peuvent être préparés par le procédé décrit dans le brevet US 3 856 708.

De manière semblable, des billes à porosité contrôlée peuvent être obtenues par addition de porogène et agglomération dans un bol tournant ou drageoir ou par le procédé 'Oil-drop'.

Selon une autre caractéristique de l'invention, les particules de catalyseur présente une surface spécifique supérieur à 10 m²/g et un volume poreux égal ou supérieur à 10 ml/100 g, le volume poreux correspondant aux pores de diamètre supérieur à 500 Å étant supérieur ou égal à 10 ml/100 g.

Selon une autre caractéristique de l'invention, les particules de catalyseur présentent une surface spécifique supérieure à 50 m²/g.

Avantageusement, elles présentent un volume poreux total supérieur ou égal à 15 ml/100g avec un volume poreux correspondant aux pores de diamètre supérieur à 200 Å, supérieur ou égal à 15 ml/100g, de préférence supérieur ou égal à 20ml/100g.

Ces catalyseurs particulaires peuvent également comprendre au moins un élément choisi dans la liste consistant en le silicium, le titane, le zirconium, le vanadium, le niobium, le tantale, le tungstène, le molybdène, le fer, les terres rares ou être obtenu par dépôt et/ou adsorption sur le support d'au moins un composé oxygéné d'au moins un élément choisi dans le groupe consistant en les éléments appartenant aux groupes 1 à 16 de la classification universelle des éléments ( nouvelle classification), cette liste incluant également les terres rares. Ces éléments ou composés sont déposés ou adsorbés sur le catalyseur particulaire.

Dans le mode opératoire comprenant un catalyseur particulaire poreux supportant des composés oxygénés d'éléments, ces éléments sont avantageusement choisis dans la liste comprenant le silicium, le titane, le zirconium, le vanadium, le niobium, le tantale, le tungstène, le molybdène, le phosphore, le bore, le fer, les alcalins, les alcalino-terreux, les terres rares. Le composé oxygéné est avantageusement un oxyde simple ou mixte de un ou plusieurs des éléments cités ci-dessus. Dans ce mode de réalisation, le catalyseur poreux est de préférence un oxyde d'aluminium. Avantageusement, cette alumine présente les caractéristiques de surface spécifique et distribution de pores définis précédemment.

La concentration pondérale en composé oxygéné supporté sur un support poreux est avantageusement comprise entre 1000 ppm et 30% exprimée en masse d'élément du composé oxygéné par rapport à la masse totale du catalyseur. Cette concentration est plus préférentiellement comprise entre 0,5 % et 15 % en poids.

Quand les supports poreux correspondent à des alumines conformes à l'invention, celles-ci sont obtenues généralement par déshydratation de gibbsite, bayerite, nordstandite ou de leurs différents mélanges. Les différents procédés de préparation des alumines sont décrits dans l'encyclopédie KIRK-OTHMER, volume 2, pages 291 - 297.

On peut préparer les alumines mises en oeuvre dans le présent procédé par mise en contact d'une alumine hydratée, sous forme finement divisée, avec un courant de gaz chaud à une température comprise entre 400°C et 1000°C, puis maintien du contact entre l'hydrate et les gaz pendant une durée allant d'une fraction de seconde jusqu'à 10 secondes et enfin séparation de l'alumine partiellement déshydratée et des gaz chauds. On peut notamment se référer au procédé décrit dans le brevet américain US 2 915 365.

On peut également procéder à l'autoclavage d'agglomérés d'alumines obtenues précédemment, en milieu aqueux, éventuellement en présence d'acide, à une température supérieure à 100°C et de préférence comprise entre 150°C et 250°C, pendant une durée de préférence comprise entre 1 et 20 heures, puis à leur séchage et leur calcination.

La température de calcination est réglée de telle façon que l'on obtienne des surfaces spécifiques et des volumes poreux situés dans les zones de valeurs indiquées précédemment.

Les catalyseurs de l'invention ont avantageusement une surface spécifique supérieure à 50 m²/g. En outre, ils présentent avantageusement des pores de diamètre supérieur à 0,1µm, le volume poreux apporté par ces pores étant supérieur ou égal à 5ml/100g, avantageusement supérieur ou égal à 10ml/100g.

Dans un mode de réalisation préféré de l'invention, ces catalyseurs comprennent également des pores de diamètre égal ou supérieur à 0,5 µm, le volume poreux correspondant étant égal ou supérieur à 5 ml/100 g, de préférence supérieur ou égal à 10 ml/100 g.

Ce volume poreux généré par les pores de diamètre supérieur à 500 Å, de préférence supérieur à 0,1 µm et avantageusement supérieur à 0,5 µm permet d'obtenir des catalyseurs à durée de cycle élevée en tant que catalyseur de la réaction de cyclisation d'esters ou d'amides d'acide 6-aminocaproïque en lactames. Ainsi, de tels catalyseurs peuvent être utilisés dans des procédés industriels de production de lactames.

Selon l'invention, les catalyseurs comprenant des composés oxygénés supportés par un catalyseur poreux sont obtenus, généralement par imprégnation du catalyseur, notamment de l'alumine, par une solution d'un sel ou composés des éléments cités précédemment, puis séchés et calcinés à une température égale ou supérieure à 400°C, pour transformer éventuellement et avantageusement lesdits composés ou sels en composés oxygénés, de préférence en oxydes. Les oxydes sont déposés à la surface des pores du catalyseur poreux.

Dans un autre mode de réalisation, les composés d'éléments peuvent être ajoutés dans le matériau constituant le catalyseur poreux avant sa mise en forme ou au cours du processus de mise en forme.

La calcination des catalyseurs imprégnés est de préférence réalisée sous atmosphère oxydante telle que l'air.

Selon encore un autre mode de réalisation de l'invention, le catalyseur peut être un phosphate métallique de formule générale :

(PO₄)ₙHₕM, (Imp)ₚ

dans laquelle :
- M représente un élément divalent, trivalent, tétravalent ou pentavalent choisi dans les groupes 2a, 3b, 4b ,5b, 6b, 7b, 8, 2b, 3a, 4a et 5a de la classification périodique des éléments ou un mélange de plusieurs de ces éléments ou M=O,
- Imp représente un composé d'imprégnation basique constitué d'un métal alcalin ou alcalino-terreux, ou de mélanges de plusieurs de ces métaux, associé à un contre-anion pour assurer la neutralité électrique,
- n représente 1, 2 ou 3,
- h représente 0, 1 ou 2,
- p représente un nombre compris entre 0 et 1/3 et correspond à un rapport molaire entre l'imprégnant Imp et l'imprégné (PO₄)ₙ Hₕ M.

Parmi les métaux des groupes 2a, 3b, 4b ,5b, 6b, 7b, 8, 2b, 3a, 4a et 5a de la classification périodique des éléments, on peut citer notamment le béryllium, le magnésium, le calcium, le strontium, le baryum, l'aluminium, le bore, le gallium, l'indium, l'yttrium, les lanthanides tels que le lanthane, le cérium, le praséodyme, le néodyme, le samarium, l'europium, le gadolinium, le terbium, le dysprosium, l'holmium, l'erbium, le thulium, l'ytterbium et le lutécium, le zirconium, le titane, le vanadium, le niobium, le fer, le germanium, l'étain, le bismuth.

Parmi les phosphates de lanthanides, on peut distinguer une première famille qui regroupe les orhophosphates de terres rares légères, également dénommées terres rares cériques, incluant le lanthane, le cérium, le praséodyme, le néodyme, le samarium et l'europium. Ces orthophosphates sont dimorphiques. Ils présentent une structure hexagonale et évoluent vers une structure monoclinique, lorsqu'ils sont chauffés à une température de 600 à 800°C.

Une deuxième famille de phosphates de lanthanides regroupe les orthophosphates de gadolinium, de terbium et de dysprosium. Ces orthophosphates présentent la même structure que les orthophosphates de terres rares cériques, mais présentent en plus une troisième phase cristalline de structure quadratique à haute température (vers 1700°C).

Une troisième famille de phosphates de lanthanides regroupe les orthophosphates de terres rares lourdes, appelées également terres rares yttriques, incluant l'yttrium, l'holmium, l'erbium, le thulium, l'ytterbium et le lutécium. Ces composés cristallisent uniquement sous la forme quadratique.

Parmi les différentes familles d'orthophosphates de terres rares précitées, on fait appel préférentiellement aux orthophosphates de terres rares cériques.

On peut mettre en oeuvre des phosphates métalliques de formule précédente qui sont des mélanges de phosphates de plusieurs des métaux indiqués précédemment ou des phosphates mixtes de plusieurs des métaux indiqués précédemment ou encore des phosphates mixtes contenant un ou plusieurs des métaux indiqués précédemment et un ou plusieurs autres métaux tels que les métaux alcalins ou alcalino-terreux.

Les contre-anions entrant dans la formule du composé d'imprégnation Imp sont basiques. On peut notamment utiliser les ions hydroxyde, phosphate, hydrogénophosphate, dihydrogénophosphate, chlorure, fluorure, nitrate, benzoate, oxalate, sans que ces citations soient limitatives.

Le rapport molaire p est de préférence compris entre 0,02 et 0,2.

Si l'on se réfère aux techniques générales de préparation de phosphates (telles que décrites notamment dans "PASCAL P. Nouveau traité de chimie minérale" tome X (1956), pages 821-823 et dans "GMELINS Handbuch der anorganischen Chemie" (8ème édition) volume 16 (C), pages 202-206 (1965), on peut distinguer deux voies principales d'accès aux phosphates. D'une part, la précipitation d'un sel soluble du métal (chlorure, nitrate) par l'hydrogénophosphate d'ammonium ou l'acide phosphorique. D'autre part, la dissolution de l'oxyde ou du carbonate du métal (insolubles) avec de l'acide phosphorique, généralement à chaud, suivie d'une précipitation.

Les phosphates précipités obtenus selon l'une des voies indiquées peuvent être séchés, traités par une base organique (telle que l'ammoniaque) ou minérale (telle qu'un hydroxyde de métal alcalin) et être soumis à une calcination, ces trois opérations pouvant être réalisées dans l'ordre indiqué ou dans un ordre différent.

Les phosphates métalliques de formule précédente pour lesquels le symbole p est supérieur à 0, peuvent être préparés par imprégnation du composé (PO₄)ₙ Hₙ M préparé selon l'une des techniques décrites précédemment, avec une solution ou une suspension de Imp dans un solvant volatil, tel que l'eau de préférence.

Les résultats sont d'autant meilleurs que Imp est plus soluble et que le composé (PO₄)ₙ Hₕ M est plus fraîchement fabriqué.

Ainsi un procédé avantageux de préparation de ces phosphates consiste :
a) à réaliser la synthèse du composé (P0₄)ₙ Hₕ M ; puis de préférence sans séparer (PO₄)ₙ Hₕ M du milieu réactionnel;
b) à introduire l'imprégnant Imp dans le milieu réactionnel ;
c) à séparer l'éventuel liquide résiduel du solide réactionnel ;
d) à sécher et éventuellement à calciner.

Les performances de ces catalyseurs et notamment leur résistance à la désactivation peuvent être encore améliorées par une calcination. La température de calcination sera avantageusement comprise entre 300°C et 1000°C et de préférence entre 400°C et 900°C. La durée de la calcination peut varier dans de larges limites. A titre indicatif, elle se situe généralement entre 1 heure et 24 heures.

Parmi les catalyseurs préférés dans le procédé de l'invention, on peut citer plus particulièrement le phosphate de lanthane, le phosphate de lanthane calciné, le phosphate de lanthane associé à un dérivé du césium, du rubidium ou du potassium, le phosphate de cérium calciné, le phosphate de cérium associé à un composé du césium, du rubidium ou du potassium, le phosphate de samarium associé à un composé du césium, du rubidium ou du potassium, le phosphate d'aluminium, le phosphate d'aluminium associé à un composé du césium, du rubidium ou du potassium, le phosphate de niobium calciné, le phosphate de niobium associé à un composé du césium, du rubidium ou du potassium, l'hydrogénophosphate de zirconium calciné, l'hydrogénophosphate de zirconium associé à un composé du césium, du rubidium ou du potassium.

Les orthophosphates décrits ci-dessus peuvent être utilisés en mélange avec de l'acide phosphorique (H₃PO₄).

On peut également utiliser comme catalyseur des pyrophosphates de terres rare, notamment de lanthane, seuls ou en mélange avec les orthophosphates décrits ci-dessus, de tels catalyseurs sont décrits dans le brevet européen EP1066255.

Les composés dinitriles préférés de l'invention sont des composés obtenus par hydrocyanation du butadiène et encore plus particulièrement, les composés dinitriles ramifiés produits par la double hydrocyanation du butadiène tels que le méthyl glutaronitrile, l'éthyl-succinonitrile. Avantageusement, le procédé de l'invention utilise un mélange de composés dinitriles comprenant du méthylglutaronitrile, de l'éthylsuccinonitrile et de l'adiponitrile.

Ce mélange est notamment obtenu par séparation, par exemple par distillation, à partir du milieu réactionnel obtenu après hydrocyanation des pentènenitriles, dans le procédé de production de l'adiponitrile par double hydrocyanation du butadiène.

Les alcools convenables pour l'invention sont, par exemple, les alcools aliphatiques ramifiés ou non, cycliques ou non, pouvant comporter un noyau aromatique et pouvant comprendre de 1 à 20 atomes de carbone. A titre d'exemples préférés, on peut citer les alcools suivants, méthanol, propanol, isopropanol, alcool benzylique, éthanol, n-butanol, isobutanol, pentanols, cyclohexanol, hexanol, isooctanol, 2-éthyl hexanol.

La composition ou les diesters obtenus par le procédé de l'invention peuvent être utilisés seuls ou en mélange avec d'autres solvants ou avec de l'eau sous forme de solution ou émulsion. Notamment, ils peuvent être utilisés en mélange avec les diesters des diacides linéaires cités précédemment (RPDE).

Ces composés diesters trouvent des applications comme solvant dans de nombreux domaines tels que les peintures, vernis et laques, l'industrie du revêtement de surfaces ou de tout autre article comme les câbles par exemple, l'industrie des encres, des lubrifiants pour textiles, les liants et résines pour noyaux et moules de fonderie, des produits nettoyants, les formulations cosmétiques, pour la mise en oeuvre de certaines réactions chimiques, dans les compositions de traitement des sols et végétaux et plus généralement, l'utilisation seul ou dans une formulation, comme solvant de nettoyage, décapage, dégraissage dans toute activité" industrielle ou domestique.

Ces composés diesters peuvent également être utilisés comme plastifiants de certaines matières plastiques ou comme monomères pour la fabrication de polymères.

D'autres avantages, caractéristiques de l'invention seront mieux détaillés et illustrés au vu des exemples donnés ci-après uniquement à titre illustratif.

### Synthèse des diesters en une étape (comparatif)

Un milieu comprenant de l'eau, du méthanol et un mélange de composés dinitriles de composition pondérale suivante :
➢ 86 % en poids de méthylglutaronitrile
➢ 11 % en poids d'éthylsuccinonitrile
➢ 3 % en poids d'adiponitrile.
est introduit à l'aide d'un pousse seringue selon un débit de 1 ml/h dans un tube en pyrex placé verticalement dans un four dont la température est de 300°C et balayé par un courant d'azote de 1l/h. 4 ml de catalyseur sont placés entre 2 couches de poudre de verre de volume 5 ml. L'injection se fait juste au dessus de la couche de verre supérieure, le courant d'azote entraîne les produits à travers le lit de catalyseur. En sortie de four les gaz sont condensés dans un tube placé dans un bain de glace puis analysés en Chromatographie en phase gazeuse.

Le milieu introduit a la composition molaire suivante :
- 1 mole de composés dinitriles
- 2 moles d'eau
- 8 moles de méthanol

Un essai a été réalisé en utilisant comme catalyseur de l'alumine macroporeuse commercialisée par la société PROCATALYSE sous la dénomination SCM 139 XL. Le taux de transformation des composés dinitriles est de 25 %. Le rendement en diesters est de 0.3%. On a constaté que le milieu réactionnel contient des cyanoesters correspondant à un produit intermédiaire capable d'être transformés en diesters. Le rendement en cyanoesters est de 2.4 %.

Un second essai a été réalisé en utilisant comme catalyseur un mélange de 2 mole d'orthophosphate de lanthane et une mole d'acide orthophosphorique. Le taux de transformation des composés dinitriles est de 62 %. Le rendement en diesters est de 3%. On a constaté que le milieu réactionnel contient des cyanoesters correspondant à un produit intermédiaire capable d'être transformés en diesters. Le rendement en cyanoesters est de 2 %.

Un troisième essai a été réalisé en utilisant comme catalyseur de l'oxyde de titane (anatase) .La conversion des composés dinitriles est de 78%.Le rendement est diesters est de 3%. Le milieu réactionnel contient en outre 15% de cyanoesters et 20% de mélange d'imides .

### Synthèse des diesters en 2 étapes

### Exemple 3

Sur un lit fixe catalytique composé de 4 ml d'oxyde de titane (anatase) placé entre 2 couches de 5ml de poudre de verre chauffé à 275°C et balayé par un courant d'azote de 3 l/h, on coinjecte à l'aide de 2 pousse seringues 1 ml/h de mélange de dinitriles et 1 ml/h d'eau. En sortie du réacteur , les gaz sont condensés dans une recette placée dans un bain de glace .Apres 6 h de réaction , les produits obtenus sont analysés par chromatographie en phase gazeuse .On obtient alors pour une conversion des dinitriles de 97% un rendement en mélange d'imides de 94%.

### Exemple 4

Dans un réacteur, on introduit 1 g de mélange d'imides , 10 ml de méthanol et on ajoute 0.2 g d'oxyde de titane anatase . On chauffe le mélange réactionnel sous pression autogène à 250°C pendant 5 heures. Apres refroidissement et filtration du catalyseur, le milieu est analysé par chromatographie en phase gazeuse. Pour une conversion de 90% en imides , le rendement en diester méthylique est de 60%.

### Exemple 5

Dans un réacteur, on introduit 1 g de mélange d'imides, 10 ml de propanol-1 et on ajoute 0.2 g d'oxyde de titane anatase. On chauffe le mélange réactionnel à 250°C sous pression autogène pendant 5 heures .Apres refroidissement et filtration du catalyseur le milieu réactionnel est analysé par Chromatographie en phase gazeuse. Pour une conversion en imides de 55% on obtient un rendement en diesters propyliques de 40%.

### Exemple 6

Dans un réacteur, on introduit 1 g de mélange d'imides, 10 ml de butanol-1 et on ajoute 0.2 g d'oxyde de titane anatase. On chauffe le mélange réactionnel à 250°C sous pression autogène pendant 5 heures .Apres refroidissement et filtration du catalyseur le milieu réactionnel est analysé par Chromatographie en phase gazeuse. Pour une conversion en imides de 50% on obtient un rendement en diesters butyliques de 38%.

### Exemple 7

Dans un réacteur, on introduit 1 g de mélange d'imides, 10 ml d'alcool isobutylique et on ajoute 0.2 g d'oxyde de titane anatase. On chauffe le mélange réactionnel à 250°C sous pression autogène pendant 5 heures .Apres refroidissement et filtration du catalyseur le milieu réactionnel est analysé par Chromatographie en phase gazeuse. Pour une conversion en imides de 52% on obtient un rendement en diesters isobutyliques de 40%.

### Exemple 8 en phase gazeuse

Sur un lit catalytique composé de 4 ml d'oxyde de titane (anatase) placé entre 2 couches de 5 ml de poudre de verre chauffé à 275°C et balayé par un courant d'azote de 3 l/h, on injecte une solution composée de 1 g de mélange d'imides en solution dans 8 ml de méthanol à un débit de 5 ml/h. En sortie de réacteur, les gaz sont condensés dans une recette placée dans un bain de glace. Après 6 heures de réaction , les produits obtenus sont analysés par CPG . On obtient pour une conversion des imides de 62% un rendement en diesters méthyliques de 30%.

### Exemple 9

Sur un lit catalytique composé de 4 ml d'oxyde de titane (anatase) placé entre 2 couches de 5 ml de poudre de verre chauffé à 275°C et balayé par un courant d'azote de 3 l/h, on injecte une solution composée de 1 g de mélange d'imides en solution dans 8 ml de pentanol-1 à un débit de 5 ml/h. En sortie de réacteur, les gaz sont condensés dans une recette placée dans un bain de glace. Après 6 heures de réaction , les produits obtenus sont analysés par CPG . On obtient pour une conversion des imides de 70% un rendement en diesters pentyliques de 45%.

## Revendications

1. Procédé de fabrication en deux étapes de composés diesters par réaction entre un composé imide de formule générale I suivante : 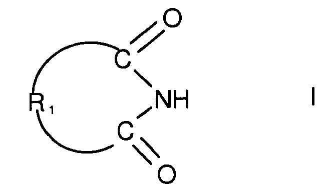 dans laquelle : R₁ représente un radical divalent hydrocarboné comprenant de 2 à 12 atomes de carbone, linéaires ou ramifiés,
avec un alcool de formule générale II suivante :
R₂-OH (II)
dans laquelle : R₂ représente un radical hydrocarboné pouvant comprendre des hétéroatomes, linéaires ou ramifiés, aliphatique, cycloaliphatique aromatique ou arylalkyl comprenant de 1 à 20 atomes de carbone,
ledit composé imide de forme générale (I) étant obtenu par hydrolyse en phase vapeur en présence d'un catalyseur solide, de composés dinitriles de formule générale III suivante :
NC-R₁-CN (III)

2. Procédé selon la revendication 1, **caractérisé en ce que** les composés dinitriles sont choisis dans le groupe comprenant le méthylglutaronitrile, l'éthylsuccinonitrile, l'adiponitrile ou leurs mélanges.

3. Procédé selon la revendication 1 ou 2, **caractérisé en ce que** l'alcool est choisi dans le groupe comprenant méthanol, propanol, isopropanol, alcool benzylique, éthanol, n-butanol, isobutanol, pentanols, cyclohexanol, hexanol, isooctanol, 2-éthyl hexanol et leurs mélanges.

4. Procédé selon l'une des revendications 1 à 3, **caractérisé en ce que** la réaction d'hydrolyse est réalisée à une température inférieure à 500°C, de préférence entre 250 et 450°C.

5. Procédé selon l'une des revendications 1 à 4 **caractérisé en ce que** le rapport molaire entre l'eau et le composé nitrile est compris entre 1 et 10 et préférentiellement compris entre 2 et 5.

6. Procédé selon l'une des revendications 1 à 5, **caractérisé en ce que** le catalyseur solide est choisi parmi les oxydes métalliques tels que l'alumine, les hétéropolyacides, les zéolithes de type pentasil et faujasite, les argiles, les phosphates métalliques, l'oxyde de titane, les mélanges silice/alumine et analogues.

7. Procédé selon la revendication 6, **caractérisé en ce que** l'argile est choisie parmi les kaolins, les serpentines, les smectites ou montmorillonites, les illites ou micas, les glauconites, les chlorites ou vermiculites, les attapulgites ou sépiolites, les argiles à couches mixtes, les allophanes ou imogolites et les argiles à haute teneur en alumine.

8. Procédé selon la revendication 7, **caractérisé en ce que** l'argile est une montmorillonite.

9. Procédé selon l'une des revendications 7 ou 8, **caractérisé en ce que** l'argile est pontée.

10. Procédé selon la revendication 6, **caractérisé en ce que** le catalyseur est un catalyseur particulaire obtenu par mise en forme d'au moins un oxyde minéral simple ou mixte d'au moins un élément choisi dans le groupe consistant en le silicium, l'aluminium, le titane, le zirconium, le vanadium, le niobium, le tantale, le tungstène, le molybdène, le fer, les terres rares, et **en ce qu'**il comprend au moins une macroporosité **caractérisé par** un volume poreux correspondant aux pores de diamètre supérieur à 500 Å, supérieur ou égal à 5ml/100g.

11. Procédé selon la revendication 10, **caractérisé en ce que** le catalyseur particulaire présente une surface spécifique supérieure à 10 m²/g et un volume poreux total supérieur ou égal à 10 ml/100 g, le volume poreux correspondant aux pores de diamètre supérieur à 500 Å étant supérieur ou égal à 10 ml/100 g.

12. Procédé selon la revendication 10 ou 11, **caractérisé en ce que** le catalyseur présente une surface spécifique supérieure à 50 m²/g.

13. Procédé selon l'une des revendications 10 à 12, **caractérisé en ce que** le catalyseur présente un volume poreux total supérieur ou égal à 20 ml/100g avec un volume poreux correspondant aux pores de diamètre supérieur à 70 Å supérieur ou égal à 20 ml/100g.

14. Procédé selon l'une des revendications 10 à 13, **caractérisé en ce que** le catalyseur particulaire est un oxyde d'aluminium.

15. Procédé selon l'une des revendications 10 à 14, **caractérisé en ce que** le catalyseur particulaire comprend au moins un élément choisi dans la liste consistant en le silicium, le titane, le zirconium, le vanadium, le niobium, le tantale, le tungstène, le molybdène, le fer, les terres rares ou au moins un composé oxygéné d'au moins un élément choisi dans le groupe consistant en les éléments appartenant aux groupes 1 à 16 de la classification universelle des éléments (nouvelle classification), cette liste incluant également les terres rares, déposé ou adsorbé sur le catalyseur particulaire en oxyde minéral simple ou mixte

16. Procédé selon la revendication 6, **caractérisé en ce que** le catalyseur est un phosphate métallique de formule générale :
(PO₄)ₙ Hₕ M, (Imp)ₚ
dans laquelle :
➢ M représente un élément divalent, trivalent, tétravalent ou pentavalent choisi dans les groupes 2a, 3b, 4b ,5b, 6b, 7b, 8, 2b, 3a, 4a et 5a de la classification périodique des éléments ou un mélange de plusieurs de ces éléments ou M=O,
➢ Imp représente un composé d'imprégnation basique constitué d'un métal alcalin ou alcalino-terreux ou de mélanges de plusieurs de ces métaux, associé à un contre-anion pour assurer la neutralité électrique,
➢ n représente 1, 2 ou 3,
➢ h représente 0, 1 ou 2,
➢ p représente un nombre compris entre 0 et 1/3 et correspond à un rapport molaire entre l'imprégnant Imp et l'imprégné (PO4)n Hh M.

17. Procédé selon la revendication 6, **caractérisé en ce que** le catalyseur est un pyrophosphate de terres rares

18. Procédé selon la revendication 6, 16 ou 17, **caractérisé en ce que** le catalyseur est un mélange de pyrophosphate de terre rare et d'orthophosphate de terre rare.

19. Procédé selon l'une des revendications 6 ou 16, **caractérisé en ce que** le catalyseur est un mélange d'orthophosphate de terre rare et d'acide phosphorique.

## Patentansprüche

1. Verfahren zum Herstellen von Diesterverbindungen durch Reaktion zwischen einer Imidverbindung der folgenden allgemeinen Formel I in zwei Schritten, 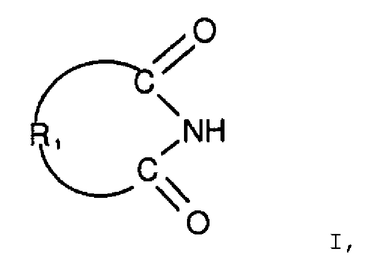 wobei R₁ einen linearen oder verzweigten zweiwertigen Kohlenwasserstoffrest darstellt, der 2 bis 12 Kohlenstoffatome umfasst,
mit einem Alkohol der folgenden allgemeinen Formel II:
R₂-OH (II),
wobei R₂ einen linearen oder verzweigten aliphatischen, cycloaliphatischen, aromatischen oder Arylalkyl-Kohlenwasserstoffrest darstellt, der Heteroatome umfassen kann und der 1 bis 20 Kohlenstoffatome umfasst,
wobei die Imidverbindung der allgemeinen Formel (I) durch Hydrolyse in der Gasphase in Anwesenheit eines Feststoffkatalysators aus Dinitrilverbindungen der folgenden allgemeinen Formel III erhalten werden kann:
NC-R₁-CN (III).

2. Verfahren gemäß Anspruch 1, **dadurch gekennzeichnet, dass** die Dinitrilverbindungen aus der Gruppe ausgewählt werden, die Methylglutaronitril, Ethylsuccinonitril, Adiponitril oder deren Mischungen umfasst.

3. Verfahren gemäß Anspruch 1 oder 2, **dadurch gekennzeichnet, dass** der Alkohol aus der Gruppe ausgewählt wird, die Methanol, Propanol, Isopropanol, Benzylalkohol, Ethanol, n-Butanol, Isobutanol, Pentanole, Cyclohexanol, Hexanol, Isooctanol, 2-Ethylhexanol und deren Mischungen umfasst.

4. Verfahren gemäß einem der Ansprüche 1 bis 3, **dadurch gekennzeichnet, dass** die Hydrolysereaktion bei einer Temperatur von kleiner als 500°C, vorzugsweise zwischen 250°C und 450°C durchgeführt wird.

5. Verfahren gemäß einem der Ansprüche 1 bis 4, **dadurch gekennzeichnet, dass** das molare Verhältnis zwischen Wasser und der Nitrilverbindung zwischen 1 und 10 und vorzugsweise zwischen 2 und 5 liegt.

6. Verfahren gemäß einem der Ansprüche 1 bis 5, **dadurch gekennzeichnet, dass** der Feststoffkatalysator aus Metalloxiden, wie zum Beispiel Aluminiumoxid, Heteropolysäuren, Zeolithen des Pentasil- und Faujasit-Typs, Ton, Metallphosphaten, Titanoxid, Siliziumdioxid-/Aluminiumoxid-Mischungen und dergleichen ausgewählt wird.

7. Verfahren gemäß Anspruch 6, **dadurch gekennzeichnet, dass** der Ton aus den Kaolinen, Serpentinen, Smectiten oder Montmorilloniten, Illiten oder Glimmern, Glaukoniten, Chloriten oder Vermiculiten, Attapulgiten oder Sepiolithen, Tonen mit gemischten Schichten, Allophanen oder Imogoliten und Tonen mit einem hohen Gehalt an Aluminiumoxid ausgewählt wird.

8. Verfahren gemäß Anspruch 7, **dadurch gekennzeichnet, dass** der Ton ein Montmorillonit ist.

9. Verfahren gemäß einem der Ansprüche 7 oder 8, **dadurch gekennzeichnet, dass** der Ton verbrückt ist.

10. Verfahren gemäß Anspruch 6, **dadurch gekennzeichnet, dass** der Katalysator ein Teilchenkatalysator ist, der durch Formen mindestens eines einfachen oder gemischten Mineraloxids aus mindestens einem Element ausgewählt aus der Gruppe bestehend aus Silizium, Aluminium, Titan, Zirkonium, Vanadium, Niobium, Tantal, Wolfram, Molybdän, Eisen, seltenen Erden erhalten wird, und dass er mindestens eine Makroporosität hat, die durch ein Porenvolumen gekennzeichnet ist, das größer oder gleich 5 ml/100 g ist und das Poren mit einem Durchmesser von größer als 500 Å entspricht.

11. Verfahren gemäß Anspruch 10, **dadurch gekennzeichnet, dass** der Teilchenkatalysator eine spezifische Oberfläche von größer als 10 m²/g und ein Gesamtporenvolumen von größer oder gleich 10 ml/100 g hat, wobei das Porenvolumen, das den Poren mit einem Durchmesser von größer als 500 Å entspricht, größer oder gleich 10ml/100 g ist.

12. Verfahren gemäß Anspruch 10 oder 11, **dadurch gekennzeichnet, dass** der Katalysator eine spezifische Oberfläche von größer als 50 m²/g hat.

13. Verfahren gemäß einem der Ansprüche 10 bis 12, **dadurch gekennzeichnet, dass** der Katalysator ein Gesamtporenvolumen von größer oder gleich 20 ml/100g hat, mit einem Porenvolumen, das Poren mit einem Durchmesser von größer als 70 Å entspricht und das größer oder gleich 20 ml/100 g ist.

14. Verfahren gemäß einem der Ansprüche 10 bis 13, **dadurch gekennzeichnet, dass** der Teilchenkatalysator ein Aluminiumoxid ist

15. Verfahren gemäß einem der Ansprüche 10 bis 14, **dadurch gekennzeichnet, dass** der Teilchenkatalysator mindestens ein Element, das aus der Liste, die aus Silizium, Titan, Zirkonium, Vanadium, Niobium, Tantal, Wolfram, Molybdän, Eisen, seltenen Erden besteht, ausgewählt wird, oder mindestens eine Sauerstoffverbindung mindestens eines Elements umfasst, das aus der Gruppe ausgewählt wird, die aus den Elementen besteht, die zu den Gruppen 1 bis 16 der universellen Klassifikation der Elemente (neue Klassifikation) gehören, wobei die Liste auch die seltenen Erden umfasst, das/die auf dem Teilchenkatalysator aus einem einfachen oder gemischten Mineraloxid abgeschieden oder adsorbiert wird.

16. Verfahren gemäß Anspruch 6, **dadurch gekennzeichnet, dass** der Katalysator ein Metallphosphat der allgemeinen Formel
(PO₄)n Hₕ M, (Imp)ₚ
ist, wobei:
• M ein zweiwertiges, dreiwertiges, vierwertiges oder fünfwertiges Element, das aus den Gruppen 2a, 3b, 4b, 5b, 6b, 7b , 8, 2b, 3a, 4a und 5a der periodischen Klassifikation der Elemente ausgewählt wird, oder eine Mischung aus mehreren dieser Elemente oder M=O, darstellt,
• Imp eine basische Imprägnierungszusammensetzung darstellt, die aus einem Alkali- oder Erdalkalimetall oder Mischungen aus mehreren dieser Metalle besteht, die an ein Gegenanion gebunden ist, um elektrische Neutralität sicherzustellen,
• n 1, 2 oder 3 darstellt,
• 0, 1 oder 2 darstellt,
• p eine Zahl zwischen 0 und 1/3 darstellt und einem molaren Verhältnis zwischen dem Imprägniermittel Imp und dem imprägnierten (PO4)n Hh M entspricht.

17. Verfahren gemäß Anspruch 6, **dadurch gekennzeichnet, dass** der Katalysator ein Pyrophosphat seltener Erden ist.

18. Verfahren gemäß Anspruch 6, 16 oder 17, **dadurch gekennzeichnet, dass** der Katalysator eine Mischung aus einem Pyrophosphat seltener Erden und einem Orthophosphat seltener Erden ist.

19. Verfahren gemäß einem der Ansprüche 6 oder 16, **dadurch gekennzeichnet, dass** der Katalysator eine Mischung aus einem Orthophosphat seltener Erden und Phosphorsäure ist.

## Claims

1. Method for the preparation in two steps of diester compounds by reaction between an imide compound of the following general formula I: 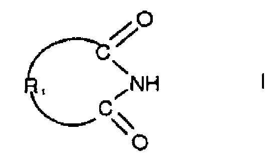 in which R₁ represents a divalent hydrocarbon radical comprising 2 to 12 carbon atoms, linear or branched,
with an alcohol of the following general formula II:
R₂-OH (II)
in which R₂ represents an aliphatic, cycloaliphatic, aromatic or arylalkyl hydrocarbon radical comprising 1 to 20 carbon atoms and which can comprise heteroatoms, linear or branched,
the said imide compound of general formula (I) being obtained by vapour phase hydrolysis, in the presence of a solid catalyst, of dinitrile compounds of the following general formula III:
**NC-R₁-CN** (III)

2. Method according to claim 1, **characterised in that** the dinitrile compounds are selected from the group comprising methylglutaronitrile, ethylsuccinonitrile, adiponitrile or their mixtures.

3. Method according to claim 1 or 2, **characterised in that** the alcohol is selected from the group comprising methanol, propanol, isopropanol, benzyl alcohol, ethanol, n-butanol, isobutanol, pentanols, cyclohexanol, hexanol, isooctanol, 2-ethylhexanol and their mixtures.

4. Method according to one of claims 1 to 3, **characterised in that** the hydrolysis reaction is performed at a temperature lower than 500°C, preferably between 250 and 450°C.

5. Method according to one of claims 1 to 4, **characterised in that** the molar ratio between the water and the nitrile compound is between 1 and 10 and preferably between 2 and 5.

6. Method according to one of claims 1 to 5, **characterised in that** the solid catalyst is selected from metal oxides such as aluminium, heteropoly acids, zeolites of the pentasil and faujasite type, clays, metal phosphates, titanium oxide, silica/aluminium mixtures and similar.

7. Method according to claim 6, **characterised in that** the clay is selected from kaolins, serpentines, smectites or montmorillonites, illites or micas, glauconites, chlorites or vermiculites, attapulgites or sepiolites, mixed layer clays, allophanes or imogolites and clays with a high aluminium content.

8. Method according to claim 7, **characterised in that** the clay is a montmorillonite.

9. Method according to one of claims 7 or 8, **characterised in that** the clay is pillared.

10. Method according to claim 6, **characterised in that** the catalyst is a particulate catalyst obtained by shaping at least one single or mixed mineral oxide of at least one element selected from the group consisting of silicon, aluminium, titanium, zirconium, vanadium, niobium, tantalum, tungsten, molybdenum, iron and the rare earths, and **in that** it comprises at least one macroporosity **characterised by** a pore volume corresponding to pores of diameter greater than 500 Å, greater than or equal to 5 ml/100 g.

11. Method according to claim 10, **characterised in that** the particulate catalyst has a specific surface area greater than 10 m²/g and a total pore volume greater than or equal to 10 ml/100 g, the pore volume corresponding to pores of diameter greater than 500 Å, being greater than or equal to 10 ml/100 g.

12. Method according to claim 10 or 11, **characterised in that** the catalyst has a specific surface area greater than 50 m²/g.

13. Method according to one of claims 10 to 12, **characterised in that** the catalyst has a total pore volume greater than or equal to 20 ml/100 g with a pore volume corresponding to pores of diameter greater than 70 Å, greater than or equal to 20 ml/100 g.

14. Method according to one of claims 10 to 13, **characterised in that** the particulate catalyst is an aluminium oxide.

15. Method according to one of claims 10 to 14, **characterised in that** the particulate catalyst comprises at least one element selected from the list consisting of silicon, titanium, zirconium, vanadium, niobium, tantalum, tungsten, molybdenum, iron and the rare earths or at least one oxygenated compound of at least one element selected from the group consisting of the elements belonging to groups 1 to 16 of the universal classification of elements (new classification), this list also including the rare earths, deposited or adsorbed on the particulate catalyst made of a single or mixed mineral oxide.

16. Method according to claim 6, **characterised in that** the catalyst is a metal phosphate of the general formula:
**(PO₄)ₙ Hₕ M, (Imp)ₚ**
in which:
➢ M represents a divalent, trivalent, tetravalent or pentavalent element selected from the groups 2a, 3b, 4b, 5b, 6b, 7b, 8, 2b, 3a, 4a and 5a of the periodic classification of elements or a mixture of a plurality of these elements or M = O,
➢ Imp represents a basic impregnation compound consisting of an alkaline or alkaline earth metal or mixtures of a plurality of these metals, associated with a counterion to ensure electrical neutrality,
➢ n represents 1, 2 or 3,
➢ h represents 0, 1 or 2,
➢ p represents a number between 0 and 1/3 and corresponds to a molar ratio between the impregnating material Imp and the impregnated material (PO4)ₙ Hh M.

17. Method according to claim 6, **characterised in that** the catalyst is a pyrophosphate of rare earths.

18. Method according to claim 6, 16 or 17, **characterised in that** the catalyst is a mixture of rare earth pyrophosphate and rare earth orthophosphate.

19. Method according to one of claims 6 to 16, **characterised in that** the catalyst is a mixture of rare earth orthophosphate and phosphoric acid.
